(19) Europäisches Patentamt
European Patent Office
Office européen des brevets

(11) **EP 1 629 856 B1**

(12) **EUROPÄISCHE PATENTSCHRIFT**

(45) Veröffentlichungstag und Bekanntmachung des Hinweises auf die Patenterteilung:
**06.08.2008 Patentblatt 2008/32**

(51) Int Cl.:
***A61M 1/28*** *(2006.01)*    ***A61K 33/14*** *(2006.01)*

(21) Anmeldenummer: **05025667.6**

(22) Anmeldetag: **31.03.2003**

(54) **Lösung für die Peritonealdialyse und sie enthaltender Doppelkammerbeutel**

Solution for peritoneal dialysis and two-chambers bag containing it

Solution pour dialyse péritonéale et sac à deux compartiments comprenant cette solution

(84) Benannte Vertragsstaaten:
**AT BE BG CH CY CZ DE DK EE ES FI FR GB GR HU IE IT LI LU MC NL PT RO SE SI SK TR**

(30) Priorität: **18.04.2002 DE 10217356**

(43) Veröffentlichungstag der Anmeldung:
**01.03.2006 Patentblatt 2006/09**

(60) Teilanmeldung:
**08011526.4**

(62) Dokumentnummer(n) der früheren Anmeldung(en) nach Art. 76 EPÜ:
**03007242.5 / 1 354 607**

(73) Patentinhaber: **Fresenius Medical Care Deutschland GmbH**
**61352 Bad Homburg (DE)**

(72) Erfinder: **Zimmeck, Thomas, Dr.**
**25551 Hohenlockstedt (DE)**

(74) Vertreter: **Herrmann, Uwe et al**
**Lorenz - Seidler - Gossel,**
**Widenmayerstrasse 23**
**80538 München (DE)**

(56) Entgegenhaltungen:
**EP-A- 0 170 275        EP-A- 0 564 672**
**EP-A- 0 602 585        EP-A- 1 008 341**
**DE-A- 19 748 290       US-A- 4 182 756**

- **PATENT ABSTRACTS OF JAPAN Bd. 1996, Nr. 09, 30. September 1996 (1996-09-30) & JP 08 131542 A (BAXTER KK), 28. Mai 1996 (1996-05-28)**

**Beschreibung**

Die vorliegende Erfindung betrifft eine Lösung für die Peritonealdialyse bestehend aus wenigstens zwei Einzellösungen, die nach einer Hitzesterilisation zusammengeführt und dem Patienten zugeführt werden, wobei die erste Einzellösung ein Osmotikum und die zweite Einzellösung einen Puffer enthält und wobei eine dieser Einzellösungen oder eine weitere Einzellösung Elektrolytsalze enthält.

Lösungen für die Peritonealdialyse enthalten im Wesentlichen drei Komponenten: das Puffersystem, Elektrolyte und ein Osmotikum. Als Osmotikum, das in osmotisch wirksamer Konzentration im Wesentlichen dazu dient, den Wassergehalt des Blutes zu verringern, wird häufig Glucose verwendet, die eine gute Osmolarität aufweist und zudem gut verträglich ist. Ein weiterer Vorteil des Einsatzes von Glucose ist der Preisvorteil gegenüber anderen in Frage kommenden Osmotika.

Ein Nachteil bei der Verwendung von Glucose besteht allerdings darin, daß diese bei der Hitzesterilisation karamelisiert, isomerisiert oder daß Abbauprodukte gebildet werden, die im Körper des Patienten schädliche Wirkungen entfalten, beispielsweise mit Proteinen weiterreagieren, was unerwünscht ist. Um diesen Nachteilen vorzubeugen, ist es aus der DE 197 48 290 A1 bekannt, eine aus zwei Einzellösungen bestehende Peritonealdialyselösung einzusetzen, wobei in der die Glucose sowie Elektrolytsalze enthaltenden Einzellösung ein pH-Wert von unter 3,2 eingestellt wird. Um bei der Mischung der Einzellösungen einen physiologisch verträglichen pH-Wert zu erhalten, wird ferner offenbart, in einer zweiten alkalischen Einzellösung außer dem in geringer Konzentration vorliegenden Bicarbonat das Salz einer schwachen Säure mit pKa < 5 vorzulegen. Diese beiden Einzellösungen werden nach der Hitzesterilisation miteinander gemischt und die Mischung wird sodann dem Patienten zugeführt. Bei geringen pH-Werten von unter 3,2 kann der Abbau von Glucose weitgehend verhindert werden.

Neben der genannten Verwendung von Glucose als Osmotikum ist es beispielsweise aus der WO 83/00087 bekannt, Glucosepolymere als Ersatz oder zusätzlich zu Glucose einzusetzen. Glucosepolymere werden insbesondere für lange Verweilzeiten in Peritonealdialyselösungen eingesetzt, weil sie ein günstiges Ultrafiltrationsprofil aufweisen. Aufgrund der im Gegensatz zu Glucose verhältnismäßig langsamen Diffusionsgeschwindigkeit von Glucosepolymeren bleibt die Osmolarität während der Behandlung im Wesentlichen erhalten. Zudem wird die Belastung der Patienten mit Glucose verringert, was insbesondere bei Diabetes-Patienten von Vorteil ist.

Der bei der Verwendung von Glucose beobachtete Abbau bei annähernd neutralen pH-Werten, insbesondere in Gegenwart von Lactat, und die Umwandlung, beispielsweise zu Fructose, Acetaldehyd und 3-Desoxyglucoson gilt in eingeschränktem Umfang auch für Glucosepolymere und Glucosepolymer-Derivate. Aus diesem Grund können Glucosepolymere bzw. Glucosepolymer-Derivat enthaltende Lösungen nicht bei neutralen pH-Werten sterilisiert werden.

Die nachfolgende Abbildung zeigt die Konzentration des Abbauproduktes 3-Desoxyglucoson für unterschiedliche das Osmotikum enthaltende, in Doppelkammerbeuteln befindliche Einzellösungen, wobei die rechts dargestellte Lösung statt Glucose ein Glucosepolymer als Osmotikum enthält. Daraus wird ersichtlich, daß auch bei der Verwendung von Glucosepolymeren verhältnismäßig große Mengen an Abbauprodukten gefunden werden. Dies ist darauf zurückzuführen, daß nicht nur die endständige Carbonylgruppe von Glucosepolymeren umgewandelt wird, sondern sich auch eine Glucoseeinheit vom Polymer abspalten kann. Darüber hinaus ist mit bislang unbekannten Umwandlungsprodukten zu rechnen, die sich noch im Polymerverbund des Osmotikums befinden.

**3-deoxyglucosone [µmol/L]**

180
160
140
120
100
80
60
40
20
0

In der bereits genannten WO 83/00087 werden Peritonealdialyselösungen beschrieben, bei denen als Osmotikum Glucosepolymere mit einem Polymerisationsgrad von wenigstens 4 eingesetzt werden. Die Peritonealdialyselösung dieser Druckschrift weist einen pH-Bereich von 5 bis 7,4 auf, was bei der Hitzesterilisation mit den oben genannten Nachteilen verbunden ist.

Will man die Probleme hinsichtlich des Abbaus bzw. der Umwandlung von Glucosepolymeren oder deren Derivate bei der Lagerung und Hitzesterilisation vermeiden, indem man den pH-Wert auf Werte von unter 3,2 einstellt, wie dies für den Fall von Glucose aus der DE 197 48 290 A1 bekannt ist, ergibt sich das Problem, daß die Polymere hydrolysiert werden, was in einem Bruch der Polymerkette bzw. in der Verringerung des mittleren Molekulargewichts resultiert. Erschwert wird die Herstellung von Glucosepolymer bzw. Glucosepolymerderivat haltigen Lösungen dadurch, daß diese möglicherweise Säuren enthalten, was bei der pH-Wert-Einstellung zu berücksichtigen ist.

Die EP 0 564 672 offenbart des weiteren eine wäßrige Peritonealdialyselösung, die aus zwei Einzellösungen erhalten wird, wobei der pH-Wert der ersten Einzellösung im Bereich zwischen 4,5 und 5,8 liegt. Die EP 0 602 585 A2 betrifft Dialyselösungen für die Peritonealdialyse, die Hydroxyethylstärke als osmotisch wirksame Substanz enthalten.

Es ist daher die Aufgabe der vorliegenden Erfindung eine Peritonealdialyselösung zur Verfügung zu stellen, die Glucosepolymer-Derivate enthält, die bei der Lagerung und der Hitzesterilisation keinem glucoseähnlichen Abbau unterliegen und auch nicht hydrolysiert werden und die einen Misch-pH-Wert im neutralen Bereich aufweisen.

Diese Aufgabe wird erfindungsgemäß dadurch gelöst, daß das Osmotikum ein Glucosepolymer-Derivat umfaßt und der pH-Wert der ersten Einzellösung im Bereich zwischen 3,5 bis 5,0 liegt. Besonders vorteilhaft ist es, wenn der pH-Wert in einem Bereich zwischen 4,0 und 4,3, vorzugsweise bei 4,2 liegt. Bei diesen pH-Werten ist annähernd kein Polymerabbau zu beobachten. Dies gilt insbesondere für einen pH-Wert von 4,0. Die 0,2 pH-Einheiten zu dem Vorzugswert von 4,2 sind als Sicherheitszuschlag für die mögliche Bildung von Säuren während der Sterilisation und Lagerung bestimmt. In dem beanspruchten pH-Bereich findet in merklichem Umfang weder die Hydrolyse des Osmotikums noch dessen glucoseähnlicher Abbau statt. Das Osmotikum kann ausschließlich durch das Glucosepolymer-Derivat gebildet werden. Denkbar ist auch, dass weitere osmotisch wirksame Substanzen enthalten sind.

In weiterer Ausgestaltung der vorliegenden Erfindung ist vorgesehen, daß es sich bei dem Glucosepolymer-Derivat um Hydroxyethylstärke (HES) handelt. Die vorliegende Erfindung bezieht sich auch auf andere derivatisierte Glucosepolymere, bei denen vorzugsweise bei der Derivatisierung nicht die freie Carbonylgruppe des Moleküls verändert wurde.

Die erste Einzellösung kann das Osmotikum, Calcium-Ionen, Magnesium-Ionen, Natrium-Ionen, $H^+$-Überschußionen und Chlorid-Ionen enthalten.

3

In bevorzugter Ausgestaltung der vorliegenden Erfindung enthält der Puffer Bicarbonat. Es handelt sich dabei um ein sehr verträgliches Puffersystem, das im basischen Bereich mit Carbonat und im sauren Bereich mit $CO_2$ im Gleichgewicht steht. Außer oder neben Bicarbonat sind auch andere Puffersysteme denkbar, die in einem physiologischen pH-Wert von ca. 7 puffern. Hierbei sind vorzugsweise Stoffe zu nennen, die im Körper leicht zu Bicarbonat abgebaut werden können. In Betracht kommen beispielsweise Lactat oder Pyruvat. Neben Bicarbonat oder anderen Puffersystemen enthält die zweite Einzellösung meist ferner NatriumIonen.

Vorteilhaft ist es, wenn die Bicarbonatkonzentration in Abhängigkeit von der Acidität der ersten Einzellösung eingestellt wird und nach der Formel: Bicarbonatkonzentration [mmol/l] = 5 x Acidität der ersten Einzellösung [mmol/l] x $V_A/V_B$ bestimmt wird, wobei $V_A$ das Volumen der ersten Einzellösung und $V_B$ das Volumen der zweiten Einzellösung darstellt.

Bei einer Acidität von 0,2 mmol/l beträgt die optimale Bicarbonatkonzentration bei gleich großen Kompartimenten eines Doppelkammerbeutels 0,5 bis 2,0 mmol/l. Dementsprechend kann die Bicarbonatkonzentration in einem Bereich liegen, dessen Untergrenze durch die Hälfte der nach Anspruch 6 bestimmten und dessen Obergrenze durch das Doppelte der nach Anspruch 6 bestimmten Bicarbonatkonzentration gebildet wird.

In weiterer Ausgestaltung der vorliegenden Erfindung ist vorgesehen, daß der Puffer das Salz einer schwachen Säure, vorzugsweise Lactat, enthält. Der pKa-Wert der schwachen Säure kann bei < 5 liegen. Es kann vorgesehen sein, daß der Puffer eine Mischung, beispielsweise aus Bicarbonat und dem Salz einer schwachen Säure, beispielsweise Lactat, enthält. Wird der Gehalt von Bicarbonat gering gehalten, beispielsweise ≤ 10 mmol/l, wie dies in der DE 197 48 290 A1 vorgeschlagen wird, hat dies den Vorteil, daß der Druck des $CO_2$ innerhalb des Aufbewahrungsbeutels gering ist, so daß keine besonderen Vorkehrungen hinsichtlich der Beutelfolie vorgesehen werden müssen. Als $CO_2$-Barriere kann hier eine übliche Polyolefinfolie eingesetzt werden .

Die erste Einzellösung kann eine physiologisch verträgliche Säure, insbesondere Salzsäure enthalten. Mit dieser läßt sich ohne weiteres der gewünschte pH-Wert-Bereich der ersten Einzellösung einstellen.

Die erste Einzellösung kann außer dem Osmotikum folgende Bestandteile aufweisen:

Natrium-Ionen [mmol/l]: 180 - 200
Calcium-Ionen [mmol/l]: 2 - 4
Magnesium-Ionen [mmol/l]: 0,8 - 1,2
$H^+$-Überschuß [mmol/l]: 0,05 - 0,1
Chlorid-Ionen [mmol/l]: 197 - 210

In weiterer Ausgestaltung der vorliegenden Erfindung ist vorgesehen, daß die Bicarbonatkonzentration der zweiten Einzellösung im Bereich zwischen 0,5 und 2,0 mmol/l, vorzugsweise bei 1,0 mmol/l liegt.

Besonders vorteilhaft ist es, wenn die erste und zweite Einzellösung in einem Doppelkammerbeutel getrennt lagerbar sind. Durch die Verwendung eines Doppelkammerbeutels ergibt sich ein besonders gutes Handling der Lösung, d. h. eine zuverlässige Trennung der beiden Einzellösungen während deren Lagerung und ein schnelles Vermischen im Bedarfsfall. Die Trennung der Einzellösungen ist sinnvoll, um zu vermeiden, daß sich bei der Verwendung von Bicarbonat als Puffer und Calcium unlösliche Niederschläge bilden. Darüber hinaus läßt sich die Reaktion der Glucosepolymer-Derivate mit Lactat als Puffersystem durch die Trennung vermeiden.

Die vorliegende Erfindung betrifft ferner einen Doppelkammerbeutel mit einer Lösung nach einem der Ansprüche 1 bis 12, der aus einem Kunststoffbeutel mit wenigstens einer ersten Kammer und einer zweiten Kammer besteht, wobei die erste Einzellösung in der ersten Kammer und die zweite Einzellösung in der zweiten Kammer aufgenommen ist. Vorteilhaft sind Mittel vorgesehen, durch die beide Kammern voneinander getrennt sind und bei deren Betätigung der Inhalt beider Kammern miteinander vermischbar ist. Hierbei kann die erste und zweite Kammer benachbart angeordnet sein. Vorzugsweise ist eine Schweißnaht vorgesehen, die die Kammern voneinander trennt und die sich bei einem Druck auf eine der Kammern öffnet. Bei entsprechender Dimensionierung öffnet sich die Schweißnaht bei Druck auf eine der flüssigkeitsgefüllten Kammern, so daß der Inhalt der beiden Kammern miteinander vermischbar ist und die Mischung schließlich dem Patienten verabreicht wird.

Im Folgenden wird ein Beispiel für die Herstellung einer erfindungsgemäßen Lösung gegeben:

Zur Herstellung der ersten Einzellösung werden in Wasser Natriumchlorid, Calciumchlorid, Magnesiumchlorid sowie ein Glucosepolymer-Derivat und Salzsäure unter Rühren in Lösung gebracht. Die Menge der zugegebenen Salzsäure wird derart eingestellt, daß der pH-Wert im Bereich von 4,1 bis 4,3, vorzugsweise bei 4,2 liegt. Während ein pH-Wert von 4,0 als ideal anzusehen ist, da hier kein Polymerabbau zu beobachten ist, dienen die 0,2 pH-Einheiten zu einem pH-Wert von 4,2 als Zuschlag für die mögliche Bildung von Säuren während der Sterilisation und Lagerung.

Die Acidität dieser ersten Einzellösung kann durch Titration mit 0,1 N NaOH bis pH 7,0 bestimmt werden.

Bei der zweiten Einzellösung wird in Wasser Natriumhydrogencarbonat unter langsamen Rühren gelöst. Die Bicarbonatkonzentration wird nach der Formel bestimmt:

$$\text{Bicarbonatkonzentration [mmol/l]} = 5 \times \text{Acidität der ersten Einzellösung [mmol/l]} \times V_A/V_B,$$

wobei $V_A$ das Volumen der ersten Einzellösung und $V_B$ das Volumen der zweiten Einzellösung darstellen.

Von dieser berechneten Bicarbonatkonzentration kann um 50 % nach unten und um 100 % nach oben abgewichen werden. Beträgt die Acidität der ersten Einzellösung beispielsweise 0,2 mmol/l und werden gleich große Kompartimente eines Doppelkammerbeutels verwendet, liegt die optimale Bicarbonatkonzentration im Bereich zwischen 0,5 und 2,0 mmol/l.

Die auf diese Weise hergestellten Einzellösungen werden anschließend durch Membransterilfilter in einen Kühltank filtriert. Nach Ansatzkontrolle und Freigabe der Lösung werden diese in einen Doppelkammermehrschichtfolienbeutel gefüllt, wobei die erste Einzellösung in eine erste Kammer und die zweite Einzellösung in eine zweite Kammer gefüllt wird. Beide Kammern sind durch eine Schweißnaht voneinander getrennt. Die Kompartimente werden jeweils mit einem Konnektor verschlossen. Anschließend wird der Doppelkammerbeutel in einen Umbeutel umverpackt und dann bei 121°C Hitze sterilisiert. Nach der Hitzesterilisation wird durch Druck auf eine der Kammern die Schweißnaht zumindest teilweise geöffnet, wonach sich die Lösungen mischen und ein Misch-pH-Wert im Bereich zwischen 6,8 und 7,0, vorzugsweise 6,8 erhalten wird.

## Patentansprüche

1.  Kombination von wenigstens zwei Einzellösungen zur Herstellung einer aus den Einzellösungen bestehenden Lösung für die Peritonealdialyse, die nach einer Hitzesterilisation zusammengeführt und einem Patienten zugeführt werden, wobei die erste Einzellösung ein Osmotikum und die zweite Einzellösung einen Puffer enthält und wobei eine dieser Einzellösungen oder eine weitere Einzellösung Elektrolytsalze enthält,
    **dadurch gekennzeichnet,**
    **dass** das Osmotikum ein Glucosepolymer-Derivat umfaßt und der pH-Wert der ersten Einzellösung in einem Bereich zwischen 3,5 bis 5,0 liegt.

2.  Kombination von wenigstens zwei Einzellösungen nach Anspruch 1, **dadurch gekennzeichnet, dass** der pH-Wert der ersten Einzellösung in einem Bereich zwischen 4,0 und 4,3, vorzugsweise bei 4,2 liegt.

3.  Kombination von wenigstens zwei Einzellösungen nach Anspruch 1 oder 2, **dadurch gekennzeichnet, dass** es sich bei dem Glucosepolymer-Derivat um Hydroxyethylstärke handelt.

4.  Kombination von wenigstens zwei Einzellösungen nach einem der Ansprüche 1 bis 3, **dadurch gekennzeichnet, dass** die erste Einzellösung das Osmotikum, Calcium-Ionen, Natrium-Ionen, Magnesium-Ionen, $H^+$-Überschußionen sowie Chlorid-Ionen enthält.

5.  Kombination von wenigstens zwei Einzellösungen nach einem der Ansprüche 1 bis 4, **dadurch gekennzeichnet, dass** der Puffer Bicarbonat enthält.

6.  Kombination von wenigstens zwei Einzellösungen nach Anspruch 5, **dadurch gekennzeichnet, dass** die Bicarbonatkonzentration in Abhängigkeit von der Acidität der ersten Einzellösung eingestellt wird und nach der Formel Bicarbonatkonzentration [mmol/l] = 5 x Acidität der ersten Einzellösung [mmol/l] x $V_A/V_B$ bestimmt wird, wobei $V_A$ das Volumen der ersten Einzellösung und $V_B$ das Volumen der zweiten Einzellösung darstellt.

7.  Kombination von wenigstens zwei Einzellösungen nach Anspruch 5, **dadurch gekennzeichnet, dass** die Bicarbonatkonzentration in einem Bereich liegt, dessen Untergrenze durch die Hälfte des nach der Formel Bicarbonatkonzentration [mmol/l] = 5 x Acidität der ersten Einzellösung [mmol/l] x $V_A/V_B$ (wobei $V_A$ das Volumen der ersten Einzellösung und $V_B$ das Volumen der zweiten Einzellösung darstellt), berechneten Wertes und dessen Obergrenze durch das Doppelte des nach dieser Formel bestimmten Wertes gebildet wird.

8.  Kombination von wenigstens zwei Einzellösungen nach einem der Ansprüche 1 bis 7, **dadurch gekennzeichnet, dass** der Puffer das Salz einer schwachen Säure, vorzugsweise Lactat, enthält.

9.  Kombination von wenigstens zwei Einzellösungen nach einem der Ansprüche 1 bis 8, **dadurch gekennzeichnet,**

**dass** die erste Einzellösung eine physiologisch verträgliche Säure, insbesondere Salzsäure enthält.

10. Kombination von wenigstens zwei Einzellösungen nach einem der Ansprüche 1 bis 9, **dadurch gekennzeichnet, dass** die erste Einzellösung außer dem Osmotikum folgende Bestandteile aufweist:

Natrium-Ionen [mmol/l]: 180 - 200
Calcium-Ionen [mmol/l]: 2 - 4
Magnesium-Ionen [mmol/l]: 0,8 - 1,2
H$^+$-Überschuß [mmol/l]: 0,05 - 0,1
Chlorid-Ionen [mmol/l]: 197 - 210

11. Kombination von wenigstens zwei Einzellösungen nach einem der Ansprüche 5 bis 10, **dadurch gekennzeichnet, dass** die Bicarbonatkonzentration der zweiten Einzellösung im Bereich zwischen 0,5 und 2,0 mmol/l, vorzugsweise bei 1,0 mmol/l liegt.

12. Kombination von wenigstens zwei Einzellösungen nach einem der Ansprüche 1 bis 11, **dadurch gekennzeichnet, dass** die erste und zweite Einzellösung in einem Doppelkammerbeutel getrennt gelagert sind.

13. Doppelkammerbeutel mit einer Kombination von wenigstens zwei Einzellösungen nach einem der Ansprüche 1 bis 12, **dadurch gekennzeichnet, dass** er aus einem Kunststoffbeutel mit wenigstens einer ersten Kammer und einer zweiten Kammer besteht, wobei die erste Einzellösung in der ersten Kammer und die zweite Einzellösung in der zweiten Kammer aufgenommen ist.

14. Doppelkammerbeutel nach Anspruch 13, **dadurch gekennzeichnet, dass** Mittel vorgesehen sind, durch die beide Kammern voneinander getrennt sind und bei deren Betätigung der Inhalt beider Kammern miteinander vermischbar ist.

15. Doppelkammerbeutel nach Anspruch 13 oder 14, **dadurch gekennzeichnet, dass** die erste und zweite Kammer benachbart angeordnet sind und durch eine Schweißnaht voneinander getrennt sind, die sich bei einem Druck auf eine der Kammern öffnet.

**Claims**

1. A combination of at least two individual solutions for the production of a solution comprising the individual solutions for peritoneal dialysis, which after a heat sterilisation operation are combined and supplied to a patient, wherein the first individual solution contains an osmotic agent and the second individual solution contains a buffer and wherein one of said individual solutions or a further individual solution contains electrolyte salts,
**characterised in that**
the osmotic agent includes a glucose polymer derivative and the pH-value of the first individual solution is in a range of between 3.5 and 5.0.

2. A combination of at least two individual solutions according to claim 1 **characterised in that** the pH-value of the first individual solution is in a range of between 4.0 and 4.3 and is preferably 4.2.

3. A combination of at least two individual solutions according to claim 1 or claim 2 **characterised in that** the glucose polymer derivative is hydroxyethyl starch.

4. A combination of at least two individual solutions according to one of claims 1 to 3 **characterised in that** the first individual solution contains the osmotic agent, calcium ions, sodium ions, magnesium ions, H$^+$ excess ions and chloride ions.

5. A combination of at least two individual solutions according to one of claims 1 to 4 **characterised in that** the buffer contains bicarbonate.

6. A combination of at least two individual solutions according to claim 5 **characterised in that** the bicarbonate concentration is adjusted in dependence on the acidity of the first individual solution and is determined in accordance with the formula bicarbonate concentration [mmol/l] = 5 x acidity of the first individual solution [mmol/l] x $V_A/V_B$,

wherein $V_A$ represents the volume of the first individual solution and $V_B$ represents the volume of the second individual solution.

7. A combination of at least two individual solutions according to claim 5 **characterised in that** the bicarbonate concentration is in a range whose lower limit is formed by half of the value calculated in accordance with the formula bicarbonate concentration [mmol/l] = 5 x acidity of the first individual solution [mmol/l] x $V_A/V_B$ (wherein $V_A$ represents the volume of the first individual solution and $V_B$ represents the volume of the second individual solution) and whose upper limit is formed by double the value calculated in accordance with that formula.

8. A combination of at least two individual solutions according to one of claims 1 to 7 **characterised in that** the buffer contains the salt of a weak acid, preferably lactate.

9. A combination of at least two individual solutions according to one of claims 1 to 8 **characterised in that** the first individual solution contains a physiologically compatible acid, in particular hydrochloric acid.

10. A combination of at least two individual solutions according to one of claims 1 to 9 **characterised in that** besides the osmotic agent the first individual solution has the following ingredients:

    sodium ions [mmol/l]: 180 - 200
    calcium ions [mmol/l]: 2 - 4
    magnesium ions [mmol/l]: 0.8 -1.2
    $H^+$ excess [mmol/l]: 0.05 - 0.1
    chloride ions [mmol/l]: 197 - 210.

11. A combination of at least two individual solutions according to one of claims 5 to 10 **characterised in that** the bicarbonate concentration of the second individual solution is in the range of between 0.5 and 2.0 mmol/l, and is preferably 1.0 mmol/l.

12. A combination of at least two individual solutions according to one of claims 1 to 11 **characterised in that** the first and second individual solutions are stored separately in a double-chamber bag.

13. A double-chamber bag with a combination of at least two individual solutions according to one of claims 1 to 12 **characterised in that** it comprises a plastic bag with at least a first chamber and a second chamber, wherein the first individual solution is received in the first chamber and the second individual solution is received in the second chamber.

14. A double-chamber bag according to claim 13 **characterised in that** there are provided means by which the two chambers are separated from each other and upon the actuation of which the content of the two chambers is miscible together.

15. A double-chamber bag according to claim 13 or claim 14 **characterised in that** the first and second chambers are arranged adjacent and are separated from each other by a weld seam which opens when a pressure is applied to one of the chambers.

**Revendications**

1. Combinaison d'au moins deux solutions individuelles pour la production d'une solution se composant des solutions individuelles pour la dialyse péritonéale, qui sont rassemblées après une stérilisation à la chaleur et sont conduites à un patient, où la première solution individuelle contient un agent osmotique et la seconde solution individuelle contient un tampon et l'une de ces deux solutions individuelles ou une autre solution individuelle contient des sels d'électrolyte,
**caractérisée en ce que**
l'agent osmotique contient un dérivé d'un polymère de glucose et la valeur du pH de la première solution individuelle est comprise entre 3,5 et 5,0.

2. Combinaison d'au moins deux solutions individuelles selon la revendication 1 **caractérisée en ce que** la valeur du pH de la première solution individuelle est comprise entre 4,0 et 3,3, est avantageusement de 4,2.

**3.** Combinaison d'au moins deux solutions individuelles selon la revendication 1 ou 2, **caractérisée en ce qu'**il s'agit, pour le dérivé d'un polymère de glucose, d'amidon d'hydroxyéthyle.

**4.** Combinaison d'au moins deux solutions individuelles selon l'une quelconque des revendications 1 à 3, **caractérisée en ce que** la première solution individuelle contient l'osmotique, des ions calcium, des ions sodium, des ions magnésium, des ions H$^+$ en excès ainsi que des ions chlorure.

**5.** Combinaison d'au moins deux solutions individuelles selon l'une quelconque des revendications 1 à 4, **caractérisée en ce que** le tampon contient du bicarbonate.

**6.** Combinaison d'au moins deux solutions individuelles selon la revendication 5, **caractérisée en ce que** la concentration en bicarbonate est ajustée selon l'acidité de la première solution individuelle et est déterminée selon la formule concentration en bicarbonate [mmoles/l] = 5 x acidité de la première solution individuelle [mmoles/l] x $V_A/V_B$, où $V_A$ représente le volume de la première solution individuelle et $V_B$ le volume de la seconde solution individuelle.

**7.** Combinaison d'au moins deux solutions individuelles selon la revendication 5, **caractérisée en ce que** la concentration en bicarbonate se trouve dans une plage dont la limite inférieure est formée par la moitié de la valeur calculée selon la formule concentration en bicarbonate [mmoles/l] = 5 x acidité de la première solution individuelle [mmoles/l] x $V_A/V_B$, où $V_A$ représente le volume de la première solution individuelle et $V_B$ le volume de la seconde solution individuelle et sa limite supérieure est formée du double de la valeur déterminée selon cette formule.

**8.** Combinaison d'au moins deux solutions individuelles selon l'une quelconque des revendications 1 à 7, **caractérisée en ce que** le tampon contient le sel d'un acide faible, avantageusement de lactate.

**9.** Combinaison d'au moins deux solutions individuelles selon l'une quelconque des revendications 1 à 8, **caractérisée en ce que** la première solution individuelle contient un acide physiologiquement compatible, en particulier un acide de sel.

**10.** Combinaison d'au moins deux solutions individuelles selon l'une quelconque des revendications 1 à 9, **caractérisée en ce que** la première solution individuelle, outre l'agent osmotique, contient les constituants suivants :

> Ion sodium [mmoles/l] : 180 - 200
> Ion calcium [mmoles/l] : 2 - 4
> Ion Magnésium [mmoles/l] : 0,8 - 1,2
> H$^+$ en excès [mmole/l] : 0,05 - 0,1
> Ions chlorure [mmoles/l] : 197 - 210

**11.** Combinaison d'au moins deux solutions individuelles selon l'une quelconque des revendications 5 à 10, **caractérisée en ce que** la concentration en bicarbonate de la seconde solution individuelle est comprise entre 0,5 et 2,0 mmoles/l avantageusement est de 1,0 mmole/l.

**12.** Combinaison d'au moins deux solutions individuelles selon l'une quelconque des revendications 1 à 11, **caractérisée en ce que** la première et la seconde solution individuelle sont logées séparément dans un sac à deux compartiments.

**13.** Sac à deux compartiments avec une combinaison d'au moins deux solutions individuelles selon les revendications 1 à 12, **caractérisé en ce qu'**il se compose d'un sac en matière synthétique avec au moins un premier compartiment et un second compartiment, et la première solution individuelle est reçue dans le premier compartiment et la seconde solution individuelle est reçue dans le second compartiment.

**14.** Sac à deux compartiments selon la revendication 13, **caractérisé en ce que** des moyens sont prévus par lesquels les deux compartiments sont séparés l'un de l'autre et par l'actionnement desquels, le contenu des deux compartiments se trouve mélangé.

**15.** Sac à deux compartiments selon la revendication 13 ou 14, **caractérisé en ce que** le premier et le second compartiment sont placés côte-à-côte et sont séparés par une ligne de soudure qui s'ouvre par une pression sur l'un des compartiments.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 19748290 A1
- WO 8300087 A
- EP 0564672 A
- EP 0602585 A2